(19) Europäisches Patentamt
European Patent Office
Office européen des brevets

(11) Publication number: 0 164 635
A2

# (12) EUROPEAN PATENT APPLICATION

(21) Application number: 85106521.9

(22) Date of filing: 28.05.85

(51) Int. Cl.[4]: C 07 D 513/04
A 61 K 31/425
//(C07D513/04, 277:00, 235:00)

(30) Priority: 06.06.84 IT 2127484

(43) Date of publication of application: 18.12.85 Bulletin 85/51

(84) Designated Contracting States: AT BE CH DE FR GB IT LI LU NL SE

(71) Applicant: MEDIOLANUM FARMACEUTICI s.r.l.
Via S.G.Cottolengo, 23/12 e 31
Milan(IT)

(72) Inventor: Andreani, Aldo
Via C. Colombo, 49
I-20090 Trezzano Sul Naviglio (Milano)(IT)

(72) Inventor: Mascellani, Giuseppe
Via C. Colombo, 49
I-20090 Trezzano Sul Naviglio (Milano)(IT)

(72) Inventor: Rambaldi, Mirella
Via C. Colombo, 49
I-20090 Trezzano Sul Naviglio (Milano)(IT)

(72) Inventor: Rugarli, Pierluigi
Via C. Colombo, 49
I-20090 Trezzano Sul Naviglio (Milano)(IT)

(74) Representative: Bianchetti, Giuseppe
Studio Consulenza Brevettuale Via Senato 24
I-20121 Milan(IT)

## (54) Imidazothiazolemethylene-acetohydrazide derivatives, preparation thereof and pharmaceutical composition having diuretic activity.

(57) Compounds of formula I

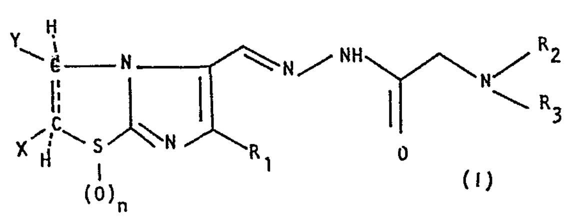

(I)

wherein

X, Y, n, $R_1$, $R_2$, $R_3$ may have the following meanings, in all the possible combinations:

X and Y, which may be the same or different, represent H, $CH_3$, $C_1$-$C_6$ alkyl groups, optionally substituted, aryl groups;

n is 0, 1, 2;

$R_1$ represents Cl, F, Br, $C_1$-$C_6$ alkyl groups, aryl and alkyl-aryl groups optionally substituted;

$R_2$ and $R_3$, which may be the same or different represent H, $CH_3$, alkyl groups, cycloalkyl groups, aryl-alkyl groups containing heteroatoms, heterocyclic groups optionally benzo-condensed;

prepared from substituted amino-acetohydrazides and imidazo (2,1-b)-thiazole-5-carboxyaldehydes, have remarkable diuretic and antihypertensive activities.

EP 0 164 635 A2

# IMIDAZOTHIAZOLEMETHYLENE-ACETOHYDRAZIDE DERIVATIVES, PREPARATION THEREOF AND PHARMACEUTICAL COMPOSITIONS HAVING DIURETIC ACTIVITY

The present invention relates to imidazothiazolemethylene-acetohydrazides having general formula (I):

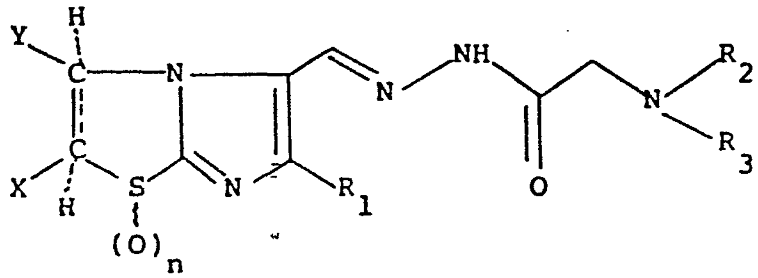

(I)

wherein X, Y, n, $R_1$, $R_2$, $R_3$ may have the following meanings, in all the possible combinations:

X and Y, which may be the same or different, represent H, $CH_3$, $C_1$-$C_6$ alkyl groups, optionally substituted, aryl groups;

n is 0, 1, 2;

$R_1$ represents Cl, F, Br, $C_1$-$C_6$ alkyl groups, aryl and alkyl-aryl groups optionally substituted;

$R_2$ and $R_3$, which may be the same or different represent H, $CH_3$, alkyl groups, cycloalkyl groups, aryl-alkyl groups containing heteroatoms, heterocyclic groups optionally benzo-fused.

The present invention relates also to the single separate isomers of said compounds, and to their pharmaceutically acceptable acid addition salts.

Compounds (I) have activity on the cardiovascular system, showing a particularly remarkable diuretic activity, better than that of some well known diuretic drugs, antihypertensive activity and a very low toxicity. Moreover, the diuretic effect being mainly achieved through the elimination of water and such electrolytes as sodium and chlorine and much less through the elimination of potassium, makes said compounds suitable for use in therapy, avoiding the risk of hypokaliemia.

The combination of the diuretic and antihypertensive effects, never attained in a single compound, is particularly useful and therapeutically advantageous in the treatment of essential hypertension, which requires prolonged administration of well tolerated or anyway low toxic drugs.

The known structurally closest compounds to those of the present invention are imidazole derivatives having a variously substituted nitrile or hydroxymethyl or carboxy group at the 5-position.

Said compounds have already been disclosed as having other types of activities (Andreani et al., Eur. J. Med. Chem. Clin. Ther. 17, 271 (1982)).

The present invention further relates to the process for preparing compounds of formula (I), starting from intermediates (II), whose preparation has been described by Andreani et al. in Farmaco ed. Sci. 37, 573, 1980.

The intermediates of formula (II) wherein one of the X or Y groups is methyl and the other one is hydrogen, are novel and therefore fall within the

scope of the present invention.

Said compounds (II) are preferably reacted with amino-acetohydrazides (III) to give imidazo/2,1-b/-thiazolemethylene-acetohydrazide compounds (I), which may be optionally oxydized to sulfonium salts or sulfoxides.

Amino-acetohydrazides (III) are preferably obtained by hydrazinolysis of the corresponding α-aminoacids methyl esters (IV) (see Scheme below).

SCHEME

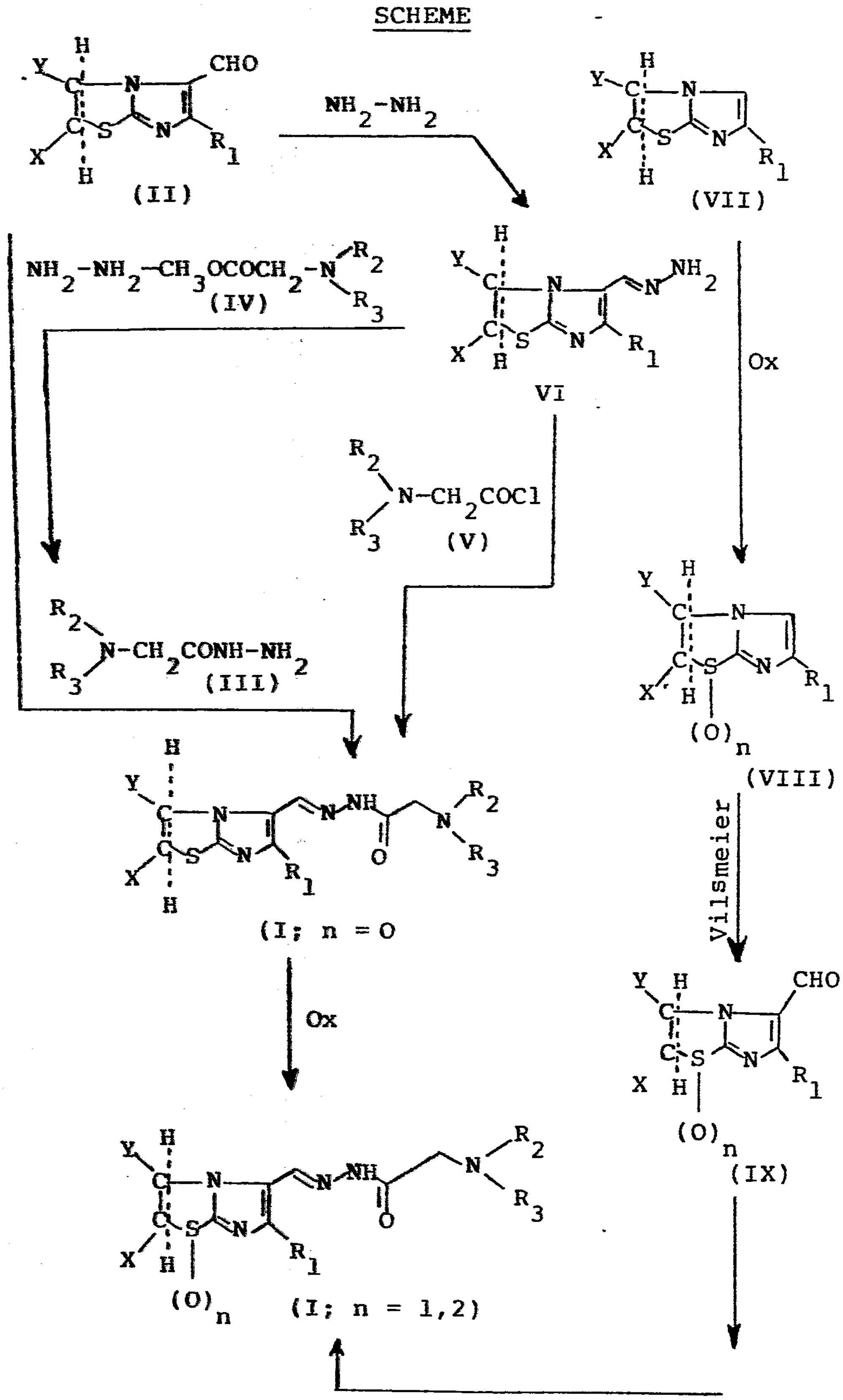

$NH_2-NH_2$
$NH_2-NH_2-CH_3OCOCH_2-N(R_2)(R_3)$ (IV)
(II)
(VII)
VI
Ox
$R_2R_3N-CH_2COCl$ (V)
$R_2R_3N-CH_2CONH-NH_2$ (III)
(VIII)
Vilsmeier
(I; n = O)
Ox
(IX)
(I; n = 1,2)

Alternatively, compounds of general formula (I) may be obtained by reacting a N,N-disubstituted α-aminoacid acyl halide (V) with 5-hydrazomethylene-imidazo/2,1-b/-thiazoles of formula (VI), formed by condensing hydrazine and 5-formyl-imidazo-thiazoles (II).

The sulfoxides or sulfones of the compounds of the invention may obtained by oxydation with peracetic acid or hydrogen peroxyde or lead tetraacetate, from the corresponding compounds (I) in which n = 0.

An alternative route to the above mentioned one to obtain sulfoxides and sulfones consists in the oxydation of imidazo/2,1-b/-thiazoles of general formula (VII), with suitable oxydizing agents, thus preparing compounds (VIII) which give, through a Vilsmeier's reaction, the corresponding 5-formyl-derivatives of general formula (IX), from which compounds of formula (I) wherein n = 1,2 may be prepared through one of the above described processes.

EXAMPLE 1

N,N-Dimethylamino-acetyl-//6-chloro-imidazo/2,1-b/-thiazole-5-yl/methylene/hydrazide

1.86 grams of 5-formyl-6-chloro-imidazo/2,1-b/-thiazole (0.01 mole) (J. Med. Chem., 12, 1031 (1969)) were dissolved in 10 ml of ethanol. 1.7 Grams (0.011 mole) of N,N-dimethylamino-acetohydrazide hydrochloride dissolved in the minimum amount of water, was added.

The reaction mixture was heated to reflux for 15 minutes, then cooled and the acetohydrazone

hydrochloride was precipitated. By dissolution in water and alkalinization with ammonia N,N-dimethylamino-acetyl/[6-chloro-imidazo[2,1-b]- thiazole-5-yl]-methylene]hydrazide precipitated. By crystallization from ethanol and drying 2.18 g (76.3% yield) of product were obtained, m.p. 183-185°C.

**EXAMPLES 2-5**

The procedure of Example 1 was repeated, using suitable reagents and making non critical changements non critical for the reaction conditions, to obtain compounds 2-5 whose characteristics are reported in Table I.

**EXAMPLE 6**

a) 2-Methyl-6-methyl-imidazo[2,1-b]thiazole

4 Grams of 2-amino-5-methylthiazole (0.035 mole) (Gazz. Chim. ital., 97, 488 (1967)) dissolved in 60 ml of chloroform were treated with 6.87 g of bromoacetone (0.05 mole). The resulting precipitate, consisting of non cyclized 2,3-dihydro-2-imino-3-acetylmethyl-5-methyl-thiazole hydrobromide, was filtered, poured in 50 ml of normal hydrobromic acid and refluxed for 0.5 hours to cyclize to 2-methyl-6-methyl-imidazo[2,1-b]thiazole hydrobromide.

The reaction mixture was treated with ammonia to markedly alkaline pH and the product was extracted with chloroform (3 x 50 ml). The collected chloroform layers were dried over sodium sulfate and evaporated to dryness. 3.96 Grams of 2-methyl-6-methyl-imidazo[2,1-b]thiazole, in form of an oil was obtained (74.5% yield).

IR (nujol) $cm^{-1}$ 1600, 1280, 1150, 680.

b) 2-Methyl-5-formyl-6-methyl-imidazo/2,1-b/thiazole

To the Vilsmeier's reactive, prepared at low temperature from 6 ml of chloroform, 4.38 g of N,N-dimethylformamide and 9.2 g of phosphorous oxychloride, 4.56 g of compound obtained in Example 6a) dissolved in 45 ml of chloroform was added. The mixture was heated to reflux for 5 hours. Chloroform was evaporated and the mixture was poured in ice-water. Precipitation was completed by addition of ammonia till pH 6. After drying, 3.69 g of raw product (68.3%) was obtained. By crystallization from ethanol 2-methyl-5-formyl-6-methyl-imidazo/2,1-b/thiazole was obtained, m.p. 158-159°C.

Elemental analysis:

| | %C | %H | %N |
|---|---|---|---|
| Calc. | 53.31 | 4.47 | 15.55 |
| Found | 53.23 | 4.49 | 15.58 |

A procedure analogous to that of Example 1 was repeated, obtaining N,N-dimethylamino-acetyl-//2-methyl-6-methyl-imidazo/2,1-a/thiazole-5-yl/methylene/-hydrazide, which after crystallization from toluene had m.p. 177-178°C.

c) A procedure analogous to that of Example 6b) was repeated, obtaining 3-methyl-5-formyl-6-phenyl-imidazo/2,1-b/thiazole, m.p. 167-169°C, after crystallization from ethanol.

IR (nujol) $cm^{-1}$ 1665, 1340, 1270, 700.

Elemental analysis

| | %C | %H | %N |
|---|---|---|---|
| Calc. | 64.44 | 4.16 | 11.56 |
| Found | 64.04 | 4.13 | 10.98. |

b) A procedure analogous to that of Example 1 was repeated, obtaining compound 7, whose characteristics are reported in Table I.

EXAMPLE 8

a) 2-Methyl-5-formyl-6-phenyl-imidazo/2,1-b/thiazole

To the Vilsmeier's reactive prepared at low temperature from 6 ml of chloroform, 4.38 g of N,N-dimethylformamide and 9.2 g of phosphorous oxychloride, 6.43 g of 2-methyl-6-phenyl-ilidazo/2,1-b/-thiazole (Gazz. Chim. Ital., 97, 488 (1967) dissolved in 50 ml of chloroform were added.

The reaction mixture was refluxed for 5 hours, then chloroform was evaporated off and the reaction mixture was treated with ice-water, and finally with ammonia till pH 6.5. 4.98 Grams of 2-methyl-5-formyl-6-phenyl-imidazo/2,1-b/thiazole (68.5% yield) was obtained. The product crystallized from ethanol and had m.p. = 172-175°C.

IR (nujol) $cm^{-1}$ 1630, 1320, 850, 705.

Elemental analysis

| | %C | %H | %N |
|---|---|---|---|
| Calc. | 64.44 | 4.16 | 11.56 |
| Found | 64.54 | 4.05 | 11.32. |

b) N,N-Dimethylaminoacetyl-//2-methyl-6-phenyl-imidazo/2,1-b/thiazole-5-yl/methylene/hydrazide

9.69 Grams of 2-methyl-5-formyl-6-phenyl-imida-

zo/2,1-b/thiazole (0.04 mole) was dissolved in 300 ml of ethanol. 6.88 Grams of N,N-dimethylamino acetohydrazide hydrochloride (0.045 mole) dissolved in 50 ml of water was added. The solution was refluxed for 10 minutes. N,N-Dimethylamino-acetyl-//2-methyl-6-phenyl-imidazo/2,1-b/thiazole-5-yl/methylene/hydrazide hydrochloride precipitated upon cooling.

After dissolution in water and treatment with ammonia, the corresponding base was obtained, which was recovered by filtration and dried. 11.26 Grams (82.5% yield) of product was obtained, which crystallized from toluene and had m.p. = 204-205°C, whose characteristics are reported in Table I.

EXAMPLES 9-14

Analogously to the above examples, starting from suitable intermediates and making changements non critical for the operative conditions, known to the expert, compounds 9-14 were obtained, whose characteristics are listed in Tables II and III.

TABLE I

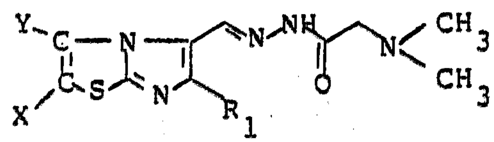

| Example n. | X | Y | $R_1$ | Rough formula | Cryst. solv. | M. P. | Elemental analysis | %C | %H | %N | IR. (nujol) $cm^{-1}$ |
|---|---|---|---|---|---|---|---|---|---|---|---|
| 1 | H | H | Cl | $C_{10}H_{12}ClN_5OS.H_2O$ | EtOH | 183-185 | Calc.<br>Found | 39.54<br>39.85 | 4.64<br>4.16 | 23.06<br>23.27 | 1675,1300,1240<br>1150 |
| 2 | H | H | $CH_3$ | $C_{11}H_{15}N_5OS$ | EtOH | 174-175 | | 49.79<br>49.53 | 5.70<br>5.58 | 26.40<br>26.10 | 1675,1610,1300<br>1260 |
| 3 | H | H | $C_6H_5$ | $C_{16}H_{17}N_5OS$ | EtOH | 160-161 | | 58.69<br>58.80 | 5.23<br>5.62 | 21.40<br>20.98 | 1670,1285,1255<br>1150 |
| 4 | H | H | $4-Cl-C_6H_4$ | $C_{16}H_{16}ClN_5OS$ | EtOH | 225-227 | | 53.10<br>53.12 | 4.46<br>4.47 | 19.35<br>19.10 | 1675,1290,1235<br>1150 |
| 5 | H | H | $4-CH_3-C_6H_4$ | $C_{17}H_{19}N_5OS$ | EtOH | 212-215 | | 59.80<br>60.12 | 5.61<br>5.81 | 20.51<br>20.29 | 1675,1370,1285<br>1160 |
| 6 | $CH_3$ | H | $CH_3$ | $C_{12}H_{17}N_5OS$ | Toluene | 177-178 | | 51.59<br>51.11 | 6.13<br>6.07 | 25.07<br>24.77 | 1675,1300,1250<br>1170 |
| 7 | H | $CH_3$ | $C_6H_5$ | $C_{17}H_{19}N_5OS.2H_2O$ | | 110-113 | | 54.09<br>54.22 | 6.14<br>6.10 | 18.55<br>18.80 | 1690,1570,1225<br>1155 |
| 8 | $CH_3$ | H | $C_6H_5$ | $C_{17}H_{19}N_5OS$ | Toluene | 204-205 | | 59.80<br>60.08 | 5.61<br>5.57 | 20.51<br>20.14 | 1655,1595,1530<br>1325 |

TABELLA II

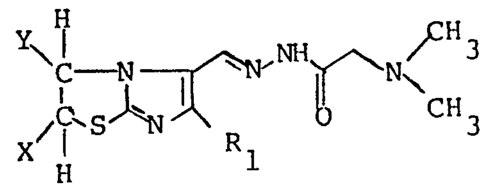

| Example n. | X | Y | $R_1$ | Rough formula | Cryst. solv. | M. P. | Elemental analysis | %C | %H | %N | IR.(nujol) $cm^{-1}$ |
|---|---|---|---|---|---|---|---|---|---|---|---|
| 9 | H | H | $C_6H_5$ | $C_{16}H_{19}N_5OS$ | Toluene | 174-175 | Calc.<br>Found | 58.33<br>58.27 | 5.81<br>5.84 | 21.26<br>21.04 | 1690,1600,<br>1300,1255 |
| 10 | H | H | $CH_3$ | $C_{11}H_{17}N_5OS$ | Toluene | 128-130 | | 49.41<br>49.84 | 6.41<br>6.48 | 26.20<br>25.79 | 1675,1610,<br>1370,1260 |
| 11 | H | H | 4-Cl-$C_6H_4$ | $C_{16}H_{18}ClN_5OS.\frac{1}{2}H_2O$ | Toluene | 187-188 | | 51.53<br>51.06 | 5.13<br>4.85 | 18.78<br>18.31 | 1690,1300,<br>1250, 830 |
| 12 | H | H | 4-$CH_3$-$C_6H_4$ | $C_{17}H_{21}N_5OS$ | Toluene | 203-204 | | 59.45<br>59.78 | 6.16<br>6.28 | 20.39<br>20.39 | 1670,1585,<br>1540,1250 |
| 13 | H | H | Cl | $C_{10}H_{14}ClN_5OS.H_2O$ | Toluene | 120-125 | | 39.34<br>39.74 | 5.28<br>4.80 | 22.94<br>23.08 | 1685,1290,<br>1240,1165 |

TABLE III

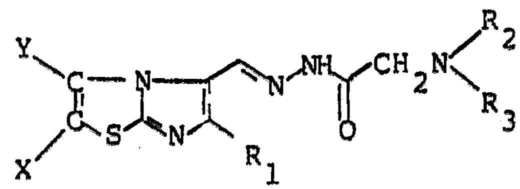

| Example n. | X | Y | $R_1$ | $R_2$ | $R_3$ | Rough formula | Cryst. solv. | M.P. | Elemental analysis %C %H %N | IR (nujol) $cm^{-1}$ |
|---|---|---|---|---|---|---|---|---|---|---|
| 14 | H | H | $C_6H_5$ | H | H | $C_{14}H_{13}N_5OS$ | EtOH | 210-215 | Calc. 56.17 4.37 23.40<br>Found 55.89 4.25 23.11 | 1675,1270,<br>1245, 955 |

## Pharmacological properties

The compounds of the invention were tested for diuretic activity and acute toxicity, in comparison with the known drugs furosemide, spironolactone, acetazolamide and hydroflumethiazide.

The first series of tests was carried out to rats orally administered with distilled water (25 ml/kg) containing the product under test, previously suspended in Tween 80 (1% v/v with respect to water). Urina of the 6 hours following the treatment was collected in order to measure the sodium concentration, by means of atomic absorption. The ratio of the meq/100 g of body weight of the treated animals to the corresponding ratio of the meq/100 g of body weight of the control animals (treated with the sole vehicle) was calculated, in order to determine the dose of the test compound doubling or trebling or anyway increasing the sodium excretion.

Table IV summarizes the values of diuretic activity and toxicity.

TABLE IV: DIURETIC ACTIVITY

ACTIVITY (I)

| Compound example n. | Dose (mg/kg) | Diuretic effect % | $DL_{50}$ in the rat (mg/kg) |
|---|---|---|---|
| 7 | 0.5 | 250 | $>$ 3000, os and i.p. |
| 8 | 1 | 300 | $>$ 3000, os and i.p. |
| 3 | 5 | 310 | ~ 600, os and i.p. |
| 5 | 5 | 130 | $>$ 3000, os and i.p. |
| 9 | 10 | 230 | $>$ 3000, os<br>$>$ 1000, i.p. |
| 11 | 20 | 230 | ~ 800, os<br>~ 600, i.p. |
| 4 | 20 | 170 | $>$ 1000, os and i.p. |
| 1 | 20 | 120 | ~1500, os<br>~250, i.p. |
| 14 | 20 | 130 | $>$1000, os<br>~600, i.p. |
| 10 | | - | $>$1000, os and i.p. |
| 2 | | - | ~600 mg/kg, i.p.<br>~1500 mg/kg, os |

TABLE IV - DIURETIC ACTIVITY (follows)

| Compound example n. | Dose (mg/kg) | ACTIVITY (I) Diuretic effect % | DL$_{50}$ in the rat (mg/kg) |
|---|---|---|---|
| 12 | 20 | 120 | ~600, mg/kg, i.p.<br>~800, mg/kg, os |
| 6 | 10 | 180 | ~600, mg/kg, i.p.<br>~1000, mg/kg, os |
| Control | - | 100 | |
| Furosemide | 10 | 490 | 2600 (rat, os) |
| Hydroflumethiazide | 5 | 260 | 750 (rat, i.v.) |
| Spironolactone | 20 | 360 | |
| Acetazolamide | 2.5 | 200 | |

(I) Diuretic effect expressed as the percentage increase of sodium urinary excretion (in comparison with controls) caused by the reported dose.

Compounds of Examples 7 and 8 were also tested in the rat at two dose levels in comparison with furosemide.

Urinary volumes excreted in the periods 0-3 and 3-6 hours from the beginning of the treatment and meq/kg of excreted electrolytes were measured.

The compounds were administered by the oral route, under the same conditions as described in the previous test, but the animals, after the 0-3 hours interval, were treated with further 25 ml/kg of distilled water, by intubation. The results are reported in Tables V and VI.

TABLE V - EXCRETED URINARY VOLUMES WITH RESPECT TO BODY WEIGHT (ml/kg) IN THE RAT.

| | | TREATMENTS | | |
|---|---|---|---|---|
| TIMES | CONTROL | FUROSEMIDE 10 mg/kg | COMPOUND EX. N. 8 10 mg/kg | COMPOUND EX. N .8 2.5 mg/kg |
| 0-3 h | 20.3 | 36.5 | 34.5 | 26.5 |
| 3-6 h | 10.5 | 10.7 | 16.4 | 16.7 |
| 0-6 h | 30.8 | 47.2 | 50.9 | 43.2 |

| | | TREATMENTS | | |
|---|---|---|---|---|
| TIMES | CONTROL | FUROSEMIDE 10 mg/kg | COMPOUND EX. N. 7 10 mg/kg | COMPOUND EX. N. 7 2.5 mg/kg |
| 0-3 h | 18.4 | 26.5 | 32.6 | 33.3 |
| 3-6 h | 17.8 | 18.6 | 14.7 | 13.6 |
| 0-6 h | 36.2 | 45.1 | 47.4 | 46.9 |

TABLE VI - EXCRETED URINARY ELECTROLYTES WITH RESPECT TO BODY WEIGHT (mEq/kg) IN THE RAT

| | | TREATMENT | | | |
|---|---|---|---|---|---|
| TIMES | IONS | CONTROLS | FUROSEMIDE 10 mg/kg | COMPOUND EX. N. 8 10 mg/kg | COMPOUND EX. N. 8 10 mg/kg |
| 0-3 h | $Na^+$ | 0.24 | 1.82 | 2.24 | 1.32 |
| | $K^+$ | 0.39 | 1.02 | 0.66 | 0.48 |
| | $Cl^-$ | - | - | - | - |
| 3-6 h | $Na^+$ | 0.049 | 0.032 | 0.15 | 0.056 |
| | $K^+$ | 0.20 | 0.13 | 0.33 | 0.35 |
| | $Cl^-$ | - | - | - | - |
| 0-6 h | $Na^+$ | 0.29 | 1.85 | 2.39 | 1.38 |
| | $K^+$ | 0.59 | 1.15 | 0.99 | 0.83 |
| | $Cl^-$ | - | - | - | - |

| | | TREATMENT | | | |
|---|---|---|---|---|---|
| TIMES | IONS | CONTROLS | FUROSEMIDE 10 mg/kg | COMPOUND EX. N. 7 10 mg/kg | COMPOUND EX. N. 7 10 mg/kg |
| 0-3 h | $Na^+$ | 0.37 | 1.30 | 2.55 | 2.03 |
| | $K^+$ | 0.59 | 0.87 | 1.04 | 0.93 |
| | $Cl^-$ | 0.46 | 1.59 | 2.55 | 1.53 |
| 3-6 h | $Na^+$ | 0.036 | 0.075 | 0.16 | 0.14 |
| | $K^+$ | 0.32 | 0.24 | 0.25 | 0.33 |
| | $Cl^-$ | 0.0 | 0.0 | 0.025 | 0.0 |
| 0-6 h | $Na^+$ | 0.41 | 1.38 | 2.71 | 2.17 |
| | $K^+$ | 0.91 | 1.11 | 1.29 | 1.26 |
| | $Cl^-$ | 0.46 | 1.59 | 2.57 | 1.53 |

In view of the various data mentioned above, the compounds of the invention can be safely administered as therapeutic agents for the prevention and treatment of various forms of hypertension, including essential renal and adrenal hypertension, for the treatment of oedema of cardiac, renal and hepatic origin and generally whenever diuresis induction is desired.

CLAIMS for the contracting States:

BE, CH, DE, FR, GB, IT, LI, LU, NL, SE

1. Compounds of general formula I

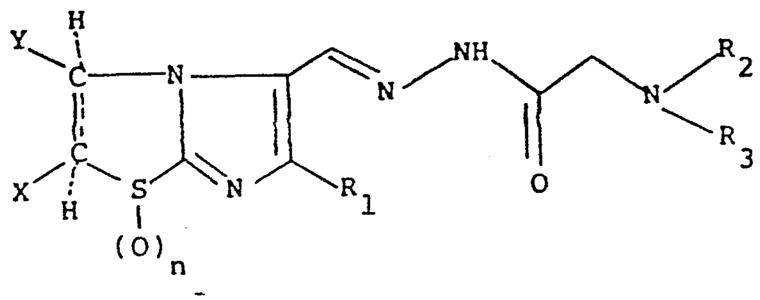

(I)

wherein X, Y, n, $R_1$, $R_2$, $R_3$ may have the following meanings, in all the possible combinations:

X and Y, which may be the same or different, represent H, $CH_3$, $C_1$-$C_6$ alkyl groups, optionally substituted, aryl groups;

n is O, 1, 2;

$R_1$ represents Cl, F, Br, $C_1$-$C_6$ alkyl groups, aryl and alkyl-aryl groups optionally substituted;

$R_2$ and $R_3$, which may be the same or different represent H, $CH_3$, alkyl groups, cycloalkyl groups, aryl-alkyl groups containing heteroatoms, heterocyclic groups optionally benzo-fused;

isomer and non-toxic, phisiologically acceptable salts.

2. Compounds according to claim 1, wherein X and Y are hydrogen, $R_1$ is a phenyl group or a phenyl group substituted by an halogen or methyl group, $R_2$ and $R_3$ represent methyl.

3. Compounds according to claim 1, wherein X is hydrogen and Y is methyl or vice versa, $R_1$ is a phenyl group or a phenyl group substituted by an halogen or methyl group, $R_2$ and $R_3$ represent methyl.

4. Compounds according to claim 1 wherein $R_1$ represent an halogen group.

6. N,N-Dimethylamino-acetyl-/[/[2-methyl-6-phenyl-imidazo/[2,1-b/]thiazol-5-yl/]methylen/]hydrazide.

7. N,N-Dimethylamino-acetyl-/[/[2,3-dihydro-6-phenyl-imidazo/[2,1-b/]thiazol-5-yl/]methylen/]hydrazide.

8. Process for the preparation of compounds of formula I characterized in that intemediates of formula II

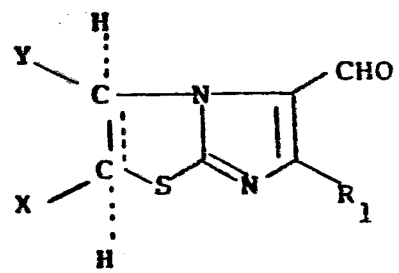

(II)

wherein X, Y, $R_1$, $R_2$ and $R_3$ have the above defined meanings, with amino-acetohydrazides (III)

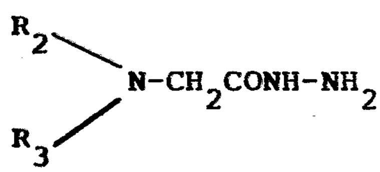

(III)

and that the obtained compounds are optionally oxidized by means of peracetic acid, hydrogen peroxide or lead tetraacetate.

9. Process for the preparation of compounds of formula I characterized by reacting intermediates of formula (IX)

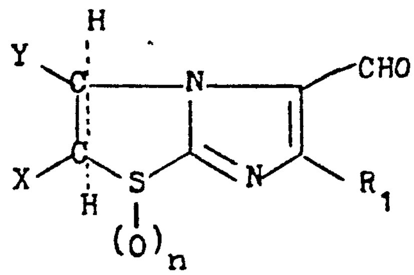

(IX)

wherein X, Y, n, R, $R_1$ and $R_3$ have the above defined meanings, with amino-acetohydrazides III.

10. As intermediates, compounds of formula II

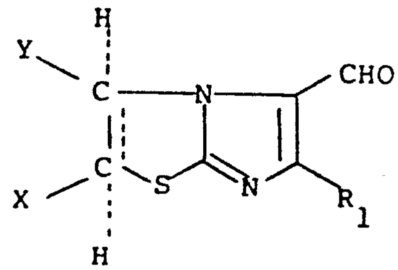

(II)

wherein X is methyl and Y is hydrogen or vice versa.

11. Pharmaceutical compositions endowed with diuretic and antihypertensive activity containing as active principle at least one of the compounds of formula I, or of its salts with physiologically acceptable acids.

CLAIMS for AT:

1. Process for the preparation of compounds of formula I

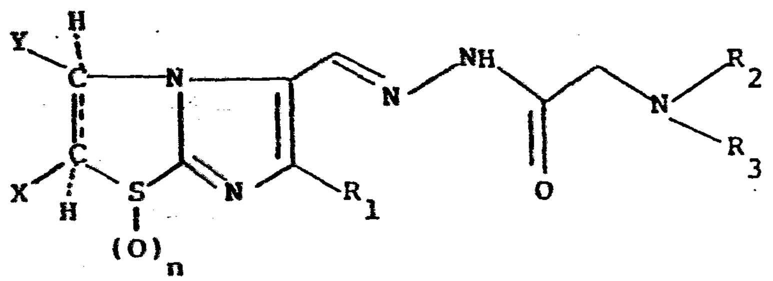

(I)

wherein X, Y, n, $R_1$, $R_2$, $R_3$ may have the following meanings, in all the possible combinations:

X and Y, which may be the same or different, represent H, $CH_3$, $C_1$-$C_6$ alkyl groups, optionally substituted, aryl groups;

n is O, 1, 2;

$R_1$ represents Cl, F, Br, $C_1$-$C_6$ alkyl groups, aryl and alkyl-aryl groups optionally substituted;

$R_2$ and $R_3$, which may be the same or different represent H, $CH_3$, alkyl groups, cycloalkyl groups, aryl-alkyl groups containing heteroatoms, heterocyclic groups optionally benzo-fused;

characterized in that intemediates of formula II

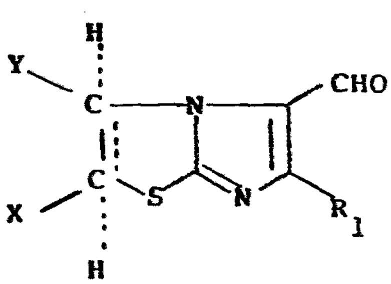

(II)

wherein X, Y, $R_1$, $R_2$ and $R_3$ have the above defined meanings, with amino-acetohydrazides (III)

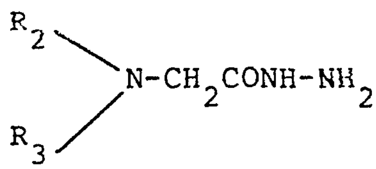

(III)

and that the obtained compounds are optionally oxidized by means of peracetic acid, hydrogen peroxide or lead tetraacetate.

2. Process for the preparation of compounds of formula I characterized by reacting intermediates of formula (IX)

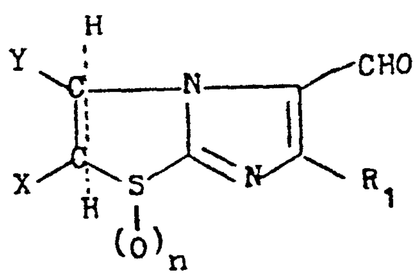

(IX)

wherein X, Y, n, R, $R_1$ and $R_3$ have the above defined meanings, with amino-acetohydrazides III.